# EUROPEAN PATENT SPECIFICATION

(11) **EP 1 883 495 B1**
(45) Date of publication and mention of the grant of the patent: **26.02.2014**
(21) Application number: 06758852.5
(22) Date of filing: 02.05.2006
(51) Int. Cl.: B23P 25/00, B23P 15/00, A61B 17/34, A61M 25/06, A61M 5/32

(54) **METHODS OF MANUFACTURING A HARD COATED CANNULA**
HERSTELLUNGSVERFAHREN FÜR HARTBESCHICHTETE KANÜLE
PROCÉDÉS DE FABRICATION D'UNE CANULE DURE REVÊTUE

(30) Priority: 02.05.2005 US 676718 P
(43) Date of publication of application: 06.02.2008
(73) Proprietor: Covidien AG, 8212 Neuhausen am Rheinfall (CH)
(72) Inventor: STEUBE, Gregory, A., St. Charles, MO 63301 (US)
(74) Representative: Tomlinson, Edward James
(86) International application number: PCT/US2006/016618
(87) International publication number: WO 2006/119182

(56) References cited:
- WO-A2-99/53974
- GB-A- 1 338 192
- US-A- 2 512 569
- US-A- 3 762 243
- US-A- 4 253 463
- US-A- 6 096 012
- US-A1- 2001 002 000
- US-A1- 2002 010 483
- US-A1- 2003 028 154
- US-A1- 2003 224 115
- US-A1- 2005 056 292
- US-B1- 6 652 546
- 'Physical vapor deposition' MCGRAW-HILL DICTIONARY OF SCIENTIFIC AND TECHNICAL TERMS. MCGRAW-HILL COMPANIES, INC. 2003, [Online] 30 August 2006, XP008074805 Retrieved from the Internet: <URL:http://www.answers.com/topic/physicalv apordeposition>

## Description

### BACKGROUND OF THE INVENTION

### 1. Field of the Invention

The subject invention relates to cannula including, but not limited to, needle cannulas, such as, for example, hypodermic needle cannulas, and more particularly, to methods of fabricating a cannula with a hard coating, such as, for example, a metallurgical-type coating, to increase point sharpness and durability, and in certain instances, the structural stiffness of cannula.

### 2. Background of the Related Art

Hypodermic needles are well known and have been widely used to deliver and withdraw fluids in the practice of medicine. For many years, hypodermic needles were treated as reusable. Thus, a medical practitioner would sharpen the needles when they became dull, and sterilize them prior to the next usage. Later, single-use disposable hypodermic needles having sharpened points were developed and they became widely used by medical practitioners.

A disposable hypodermic needle is typically formed from an elongated tube having opposed proximal and distal end portions, and a central fluid delivery lumen extending therethrough. The proximal end portion of the needle is typically associated with a fluid delivery device such as a hypodermic syringe. The distal end portion of the needle is typically provided with a pointed tip configured to pierce through an elastomeric septum and/or a patient's skin to deliver medicament held in the syringe. A hypodermic needle may also be used to aspirate fluids held in a vial. Often, such use involves inserting the pointed tip of the needle through an elastomeric seal associated with the vial to access the fluid contained therein.

There are certain considerations to address when designing the pointed tip of a hypodermic needle. For example, there is a desire to minimize the penetration force needed to urge the pointed tip of the needle through the skin of a patient. It is generally understood that a patient will experience less pain when the needle penetration force is minimized.

There have been efforts made in the prior art to address such concerns, for example, through the design of a needle point geometry that will lessen needle penetration forces. Nevertheless, there remains a need for needle points that have increased sharpness and durability, and which display reduced needle penetration forces and, thus, reduced pain or discomfort for a patient. The subject invention addresses this need by providing a novel method of fabricating a hypodermic needle with hard coated cutting edges.

In addition, the needle fabrication method disclosed herein provides advantages in other applications that are more demanding upon the needle point than typical drug injections. For example, the hard coated needles of the subject invention are well suited for injections wherein multiple medications must be withdrawn from multiple sealed vials with the same needle, or techniques such as local anesthesia infiltration, in which the patient is injected multiple times with the same needle. Hard coated needles formed in accordance with the subject invention are also advantageous in veterinary applications, for example, for injecting one or more farm animals with relatively tough hides, using the same needle.

It is widely recognized that the unsafe disposal of used hypodermic needles and syringes has lead to the spread of infectious diseases, particularly in developing countries. Consequently, efforts have been made to develop syringes that can be easily destroyed or disabled after use, so as to limit or otherwise prevent the spread of disease. Plastic syringe needles are one such development, which unlike stainless steel needles, can be easily destroyed or disabled after use to eliminate disposal concerns. Document US 25 12 569 A discloses a method of fabricating a cannula by providing an elongated thermoplastic body with a bevel and applying a metal type coating to the cannula body.

There are certain design challenges associated with the ongoing development of useful plastic hypodermic needles. In particular, the thin plastic needle must be sharp and sturdy enough to pierce through a rubber vial stopper and a patient's skin without buckling. The subject invention addresses this challenge by providing a novel method of fabricating a hard coated plastic hypodermic needle that exhibits increased sharpness, as well as structural stiffness.

### SUMMARY OF THE DISCLOSURE

The inventors of the subject invention make claim to a new and useful method of fabricating cannulas that includes, among others, the steps of providing an elongated cannula body, applying a titanium nitride coating to the elongated cannula body, and forming a bevel on a distal end portion of the elongated cannula body to fashion a sharp, durable point. Preferably, the subject invention is directed to a method of fabricating needle cannulas, such as, for example, hypodermic needle cannulas, with a coating that is measurably harder than the substrate material from which the cannula is formed.

The step of applying a titanium nitride coating to the needle body is performed prior to the step of forming the bevel on the distal end portion of the needle body. This avoids having to fixture the needle body prior to coating the finished bevel.

This method provides extremely sharp cutting edges that are harder and more durable than the underlying substrate from which the needle body is formed, which comprises a thermoplastic material. Moreover, the hard coated needle point is more resistant to damage from accidental impact or from penetrating a rubber vial plug, than the underlying substrate.

Preferably, the step of applying a titanium nitride coating to the needle body involves applying a titanium nitride coating (TiN), to an exterior surface of the needle body. Consequently, the needle body presents with an aesthetically pleasing gold colored exterior surface. In an embodiment of the subject invention, the titanium nitride coating is applied by indirect microwave heating. Other coating techniques can also be employed without departing from the scope of the subject disclosure, as claimed.

The step of forming the bevel on the distal end portion of the needle body includes forming a primary bevel surface along a primary bevel/grind plane oriented at a primary bevel/grind angle relative to the center line of the needle body, to defme a primary cutting edge at the intersection of the primary bevel surface and the exterior surface of the needle body. Preferably, this step further includes forming one or more lancets at a distal end of the bevel. The step of forming a lancet at the distal end of the bevel includes forming secondary bevel surfaces along secondary bevel/grind planes defined by an angle of rotation about the centerline and a secondary bevel/grind angle, to define secondary cutting edges at the intersections of the secondary bevel surfaces and the exterior surface of the needle body. In accordance with the subject invention, the primary and secondary cutting edges are coated with a metallurgical coating together with the exterior surface of the needle body.

These and other features of the cannula fabrication methods of subject invention will become more readily apparent from the following enabling description of the subject invention, taken in conjunction with the drawings accompanying the subject disclosure.

### BRIEF DESCRIPTION OF THE DRAWINGS

So that those having ordinary skill in the art to which the subject invention appertains will more readily appreciate how to make and use the hard coated cannula disclosed herein, preferred embodiments of the cannula, in the form of a hypodermic needle cannula and methods of fabricating the same will be described herein with respect to the drawings wherein:
Fig. 1 is a side elevational view of a disposable needle assembly that includes a hard coated hypodermic needle fabricated and constructed in accordance with a preferred embodiment of the subject invention;
Fig. 2a is an enlarged end plan view of the distal end portion of the hypodermic needle shown in Fig. 1, illustrating the primary and secondary bevel surfaces, which define the cutting edges of the needle point;
Fig. 2b is an enlarged front plan view of the distal end portion of the hypodermic needle shown in Fig. 1, illustrating the angle of rotation used to form the secondary cutting surfaces of the lancet at the distal end of the bevel; and
Fig. 2c is an enlarged side elevational view of the distal end portion of the hypodermic needle shown in Fig. 1, illustrating the angle of inclination of the primary bevel plane along which the primary bevel surface is formed.

### ENABLING DESCRIPTION OF THE PREFERRED EMBODIMENTS

The exemplary embodiments of the hard coated cannulas and the methods of fabrication disclosed herein are discussed in terms of medical needles for infusion of intravenous fluids (such as, for example, subcutaneous, intradermal and/or intramuscular delivery), medication infusion and fluid collection. It is envisioned that the present disclosure finds application to a wide variety of cannula needles, including small needle applications and devices for the infusion of preventive medications, medicaments and therapeutics to a subject.

It is also envisioned that the present disclosure may be employed for collection of body fluids including those employed during procedures relating to phlebotomy, digestive, intestinal, urinary, veterinary and the like. It is also contemplated that the hard coated needles disclosed herein may be utilized with other medical needle applications including feeding devices, phlebotomy devices, catheters, catheter introducers, guide wire introducers, spinal and epidural, biopsy, aphaeresis, dialysis, blood donor, Veress needles, Huber needles and the like.

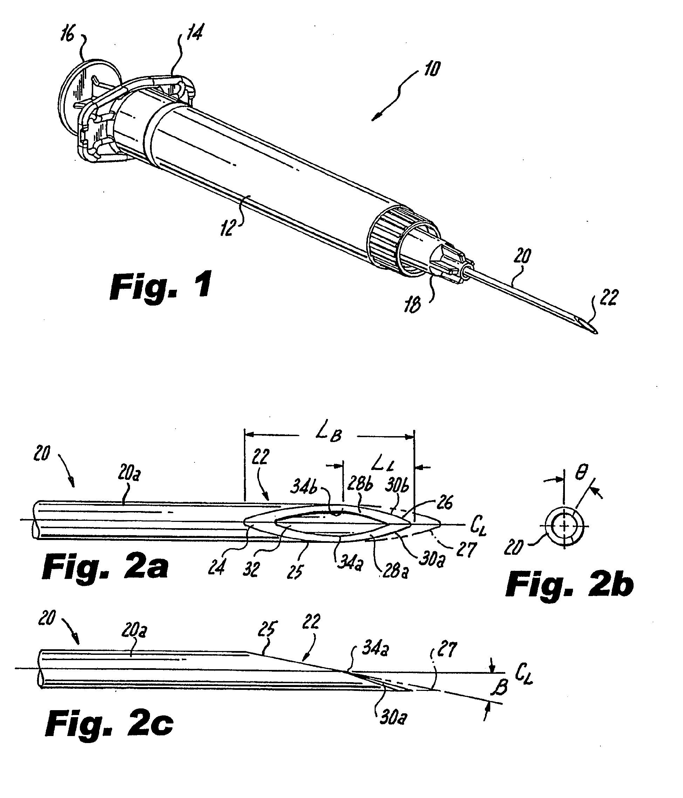

Referring now to the drawings wherein like reference numerals identify various aspects or features of the subject invention, there is illustrated in Fig. 1 a disposable needle assembly that includes a hard coated hypodermic needle cannula fabricated and configured in accordance with a preferred embodiment of the subject invention, wherein the needle assembly is designated generally by reference 10 and the hard coated hypodermic needle cannula is designated generally by reference numeral 20.

Needle assembly 10 includes an elongated tubular syringe barrel 12, which is preferably formed from a clear plastic material and which preferably includes a series of graduations imprinted thereon for accurate dosing. The proximal end portion of the syringe barrel 12 includes a pair of opposed flanges 14a, 14b to facilitate stabile handling and use of the needle assembly 10. A plunger 16 is operatively disposed within the syringe barrel 12 for facilitating the active administration of medication contained within the barrel.

A needle hub 18 is operatively associated with, and preferably detachably connected to, a distal end portion of the syringe barrel 12. The needle hub 18 mounts the hard coated hypodermic needle cannula 20 to the syringe barrel 12. More particularly, a proximal end portion of the needle cannula 20 is securely mounted within the needle hub 18. Furthermore, the needle hub 18 facilitates fluid communication between the syringe barrel 12 and the interior lumen of the hypodermic needle cannula 20. The hard coated hypodermic needle cannula 20 is provided with a well-sharpened point or bevel formed on a distal end portion of the needle body 20a that will be described in greater detail herein below.

In one example not according to the subject invention, hypodermic needle cannula 20 is preferably made from 304 stainless steel tubing, also refered to herein as tubular stock. Such tubing is typically formed by wrapping a sheet of stainless steel around a metal bar or mandel and welding the overlapping edges together to make a short, thick, solid tube. The tube is then drawn through a series of dies that reduce the diameter and wall thickness of the tubing and stretch it out into very long lengths.

In accordance with an example not of the subject invention, a metallurgical-type coating is applied to the exterior surface or outer diameter of tubular stock, before the stock is cut to form a number of hypodermic needle cannulas of given length. It is envisioned that the coating material employed may have a base of any metal, alloy, compound or composition that is suitable for application to the stainless steel substrate from which the needle body is formed, using a suitable coating technique.

Suitable metals can include, for example, chromium, molybdenum, nickel and/or cobalt. Suitable compounds can include those that combine metals with non-metals. For example, compounds that combine nitride and carbide, such as, titanium nitride or tungsten carbide, can be employed. The most preferred metallurgical coating for the hard coated hypodermic needles of the subject invention is a titanium nitride coating (TiN). The titanium nitride coating layer typically has a uniform thickness of about 3 micrometers or more and closely follows the contours of the needle body.

Preferably, the titanium nitride coating is applied to the tubular stock using a technique that involves, at least in part, indirect microwave heating. During that process, the tubular needle stock is drawn through or otherwise disposed within an insulated chamber containing plasma, which is composed of an inert gas such as argon and coating reactants. The chamber is exposed to microwave energy, which causes the reactants to disassociate from the plasma and reconstitute on the exterior surface of the tubular needle stock.

It is envisioned that other plasma coating techniques can also be employed, such as techniques that involve passing a fuel gas through a sustained electric arc to create a plasma flame. A powdered coating material is injected into the plasma flame, melted and propelled onto the surface of the article.

It is envisioned that other metallurgical coating techniques known in the art can also be employed to apply the coating to the exterior surface of the tubular stock, depending upon the coating material employed. For example, the technique of physical vapor deposition (PVD) can be employed, or certain thermal spraying techniques can be employed, such as a high velocity oxygen fuel (HVOF) process.

After the titanium nitride or a similar metallurgical coating has been applied to the surface of the tubular stock, the tubular stock is cut using conventional techniques (*e.g*., by laser or machine tools) to form a number of hypodermic needle cannulas 20 of a given length. Each hypodermic needle cannula 20 has an elongated needle body 20a with opposed proximal and distal end portions, and an interior lumen defining a central axis. After a needle cannula 20 has been cut to length, a point is ground or otherwise formed on the distal end portion of the coated needle body.

Referring to Figs. 2a through 2c, the point of the hypodermic needle cannula 20 is defined by a bevel 22 having a bevel length L_{B}. The bevel 22 includes a primary bevel surface 24 and a lancet 26 formed at the distal end of the bevel 22. The lancet 26 has a length L_{L} and includes secondary bevel surfaces 28a, 28b. A primary cutting edge 25 is defined by the intersection of the primary bevel surface 24 and the exterior surface of the tubular needle body 20a. The primary bevel surface 24 is defined where a primary bevel plane 27 meets the inner diameter of the needle body 20a. The primary bevel plane 27 is typically oriented at a relatively shallow angle relative to the centerline of the needle body. For example, the primary bevel angle β may be about between 8° to 18° relative to the centerline of the needle body. In particular, a needle for a typical drug injection (sharpest possible if desired) would typically have about 8° to 12° primary bevel angle, whereas a needle for a procedure such as nerve blocks and local anesthesia in which tactile feedback is desired to allow the physician to detect changes in the tissue layers being penetrated would typically have a blunter primary bevel angle of about 12° to 18°.

The lancet 26 is defined by two secondary bevel surfaces 28a, 28b having respective secondary cutting edges 30a, 30b formed by the intersections of the secondary bevels surfaces 28a, 28b and the exterior surface of the tubular needle body 20a. The secondary bevel surfaces 28a, 28b of lancet 26 are formed along secondary bevel planes defined by an angle of rotation θ about the centerline C_{L} of the needle body and a secondary bevel angle that is steeper than the primary bevel angle. The secondary bevel angle (not shown) is typically steeper than the primary bevel angle β and may be about between 16° to 26° relative to the centerline of the needle body. In particular, a needle for a typical drug injection (sharpest possible if desired) would typically have about a 16° to 21° secondary bevel angle, whereas a needle for a procedure such as local anesthesia would typically have a blunter secondary bevel angle of about 21° to 26°.

The angle of rotation θ that defines the secondary cutting edges can range from 25° to 65° about the central axis of the needle body. A low rotation angle will cause the lancet to have sharper, more fragile secondary cutting edges, while a high rotation angle will cause the lancet to have blunter secondary cutting edges that are less fragile. In particular, a needle for a typical drug injection (sharpest point possible) would typically have about a 25° to 35° rotation angle, whereas a needle designed for multiple use, such as a veterinary needle used for injecting animals with tough hides multiple times would benefit from a higher rotation angle of about 35° to 65°.

The primary bevel surface 24 of the bevel 22 and the secondary bevel surfaces 28a, 28b of and lancet 26, which forms part of the bevel 22, define the fluid opening 32 of the central lumen of the hypodermic needle cannula 20. The primary bevel surface 24 and the secondary bevel surfaces 28a, 28b meet at bevel intersects 34a and 34b, demarcating the primary and secondary grind planes.

Because the exterior surface of the tubular stock or the outside diameter of the hypodermic needle cannula is coated with titanium nitride or a similar metallurgical-type coating prior to cutting the cannula body to length and forming the beveled needle point, the hard coating is present on the cutting edges of the needle point formed by the respective intersections of the primary bevel surface 24 and secondary bevel surfaces 28a, 28b, with the exterior surface of the needle body 20a. More particularly, the primary cutting edge 25 of the primary bevel surface 24 and the secondary cutting edges 30a, 30b of the secondary bevel surfaces 28a, 28b forming the needle point are coated. This allows fabrication of a hypodermic needle cannula with a hard coated cutting edge without having to fixture the needle for coating after the point has been ground.

Furthermore, the titanium nitride coated hypodermic needle cannula 20 presents with an aesthetically pleasing gold colored exterior surface. While the titanium nitride coating is somewhat lubricious, it is envisioned that the hard coated hypodermic needle cannula 20 may be further coated with a biocompatible lubricant to reduce drag forces during tissue penetration or when the needle pierces through a rubber vial stopper to access fluid contained within the vial.

In accordance with another example not of the subject invention, a hypodermic needle cannula is cut to length from stainless steel tubular stock that has not been previously coated with a metallurgical-type coating. Instead, after the hypodermic needle cannula has been cut to length in a conventional manner, the needle point geometry is ground in the manner described above, so as to form a bevel having a primary bevel surface and a lancet with two secondary bevel surfaces. Thereafter, a metallurgical-type coating is applied to the exterior surface of the needle body 20a so that the primary cutting edge 25 formed by the intersection of the primary bevel surface 24 and the exterior surface of the needle body 20a, and the secondary cutting edges 30a, 30b formed by the intersection of the secondary bevel surfaces 28a, 28b and the exterior surface of needle body 20a are coated. Preferably, a titanium nitride coating is applied to the needle body by a technique that involves indirect microwave heating, as described herein above, or another known coating technique.

Those skilled in the art will readily appreciate that both of the previously described needle fabrication methods described herein provide extremely sharp cutting edges that are harder and more durable than the underlying stainless steel substrate from which the needle is formed. Moreover, the hard coated needle point is more resistant to damage from accidental impact or from penetrating a rubber vial plug, than the stainless steel substrate.

The subject invention is directed to hard coated thermoplastic cannulas, such as, for example, thermoplastic needle cannulas, and more particularly, to hard coated thermoplastic hypodermic needle cannulas and to methods of fabricating the same. Thermoplastic needle cannulas are advantageous, as compared to stainless steel needle cannulas, because they can be readily destroyed or disabled after use, for example, by exposing the needle to a flame. This is extremely useful in limiting or otherwise preventing the spread of infectious diseases, particularly in developing countries.

In one example not of the subject invention, the hard coated thermoplastic cannulas are formed by a molding process, such as, by injection molding or micro-molding. Consequently, in the case of a hypodermic needle cannula, for example, the needle point geometry (*i.e*., the primary and secondary bevel surfaces) is defined or otherwise formed by the mold. In such an instance, a hard coating, such as an amorphous vacuum deposited diamond coating, or a metallurgical-type coating, such as titanium nitride or a similar metallurgical material, is applied to the molded needle body. In so doing, the exterior surface of the needle cannula body 20a, along with the primary cutting edge 25 and the secondary cutting edges 30a, 30b are coated. In contrast, the primary and secondary bevel surfaces 24, 28a, 28b themselves are not coated. The hard coating enhances the structural stiffness of the thermoplastic needle cannula body 20a and the sharpness and durability of the primary and secondary cutting edges of the bevel 22, making the thermoplastic needle cannula 20 of the subject invention well suited for drug injections.

In an embodiment of the subject invention, hard coated thermoplastic needles are formed in conjunction with an extrusion process. In such an instance, a needle body is cut to length from extruded thermoplastic needle stock that has already been coated with a metallurgical coating, namely titanium nitride. Thereafter, the needle point geometry (*i.e*., the primary and secondary surfaces of the bevel) is mechanically formed on the distal end portion of the needle body by conventional means, such as cutting or grinding. Consequently, the exterior surface of the needle body 20a along with the primary cutting edge 25 defined at the intersection of the primary bevel surface 24 and the exterior surface of the needle body 20a, and the secondary cutting edges 30a, 30b defined at the intersection of the secondary bevel surfaces 28a, 28b are coated, thereby enhancing the structural stiffness of the needle body 20a and sharpness of the beveled needle point. However, the primary and secondary bevel surfaces 24, 28a, 28b themselves are not coated.

It is envisioned that the thermoplastic needle cannulas of the subject invention can be formed from any suitable thermoplastic material, including, without limitation, polystyrene, polycarbonate and nylon. The particular material that is used, for molding or extrusion, preferably has a melting point that will allow the thermoplastic needle body to be readily destroyed or disabled when exposed to the heat of a flame, while at the same time, be able to withstand the temperatures associated with the application of the hard coating to the needle cannula body. It is also envisioned a metallurgical-type coating, namely a titanium nitride coating, would be applied to the thermoplastic needle cannula body of the subject invention through a conventional coating process, such as, for example, physical vapor deposition (PVD) or a similar coating technique.

While the devices and methods of the subject invention have been described with respect to preferred embodiments, those skilled in the art will readily appreciate that modifications and/or changes may be made thereto, without departing from the scope of the subject invention, as defined by the appended claims.

## Claims

1. A method of fabricating a cannula comprising the steps of:
a) providing an elongated thermoplastic cannula body;
b) applying a titanium nitride coating to the cannula body; and
c) forming a bevel on a distal end portion of the cannula body,
wherein the step of applying a titanium nitride coating to the cannula body is performed prior to the step of forming a bevel on a distal end portion of the cannula body.

2. A method according to Claim 1, wherein the step of applying a titanium nitride coating to the cannula body involves applying a titanium nitride coating to an exterior surface of the cannula body.

3. A method according to Claim 1, wherein the step of applying a titanium nitride coating to the cannula body involves applying a titanium nitride coating to an exterior surface of the cannula body by indirect microwave heating.

4. A method according to Claim 1, wherein the step of applying a titanium nitride coating to the cannula body involves applying a titanium nitride coating to an exterior surface of the cannula body by physical vapor deposition.

5. A method according to Claim 1, wherein the step of forming a bevel on a distal end portion of the cannula body includes forming a first cutting edge along a primary bevel plane that intersects an exterior surface of the cannula body.

6. A method according to Claim 4, wherein the step of forming a bevel on a distal end portion of the cannula body includes forming a lancet at a distal end of the bevel.

7. A method according to Claim 6, wherein the step of forming a lancet at a distal end of the bevel includes forming secondary cutting edges along secondary bevel planes that intersect an exterior surface of the cannula body.

## Patentansprüche

1. Verfahren zur Herstellung einer Kanüle, das die folgenden Schritte umfasst:
a) Bereitstellen eines länglichen thermoplastischen Kanülenkörpers,
b) Aufbringen einer Titannitridbeschichtung auf den Kanülenkörper und
c) Bilden einer Abschrägung an einem distalen Endabschnitt des Kanülenkörpers,
wobei der Schritt des Aufbringens einer Titannitridbeschichtung auf den Kanülenkörper vor dem Schritt des Bildens einer Abschrägung an einem distalen Endabschnitt des Kanülenkörpers erfolgt.

2. Verfahren nach Anspruch 1, wobei bei dem Schritt des Aufbringens einer Titannitridbeschichtung auf den Kanülenkörper eine Titannitridbeschichtung auf eine Außenfläche des Kanülenkörpers aufgebracht wird.

3. Verfahren nach Anspruch 1, wobei bei dem Schritt des Aufbringens einer Titannitridbeschichtung auf den Kanülenkörper eine Titannitridbeschichtung durch indirekte Mikrowellenerhitzung auf eine Außenfläche des Kanülenkörpers aufgebracht wird.

4. Verfahren nach Anspruch 1, wobei bei dem Schritt des Aufbringens einer Titannitridbeschichtung auf den Kanülenkörper eine Titannitridbeschichtung durch physikalische Gasphasenabscheidung (PVD) auf eine Außenfläche des Kanülenkörpers aufgebracht wird.

5. Verfahren nach Anspruch 1, wobei der Schritt des Bildens einer Abschrägung an einem distalen Endabschnitt des Kanülenkörpers das Bilden einer ersten Schneidkante entlang einer primären Abschrägungsebene, die eine Außenfläche des Kanülenkörpers schneidet, aufweist.

6. Verfahren nach Anspruch 4, wobei der Schritt des Bildens einer Abschrägung an einem distalen Endabschnitt des Kanülenkörpers das Bilden einer Lanzette an einem distalen Ende der Abschrägung aufweist.

7. Verfahren nach Anspruch 6, wobei der Schritt des Bildens einer Lanzette an einem distalen Ende der Abschrägung das Bilden von sekundären Schneidkanten entlang sekundären Abschrägungsebenen, die eine Außenfläche des Kanülenkörpers schneiden, aufweist.

## Revendications

1. Procédé de fabrication d'une canule comprenant les étapes suivantes :
a) fournir un corps de canule thermoplastique allongé ;
b) appliquer un revêtement de nitrure de titane sur le corps de canule ; et
c) former un biseau sur une partie d'extrémité distale du corps de canule,
l'étape d'application d'un revêtement de nitrure de titane sur le corps de canule étant effectuée avant l'étape de formation d'un biseau sur une partie d'extrémité distale du corps de canule.

2. Procédé selon la revendication 1, dans lequel l'étape d'application d'un revêtement de nitrure de titane sur le corps de canule implique l'application d'un revêtement de nitrure de titane sur une surface extérieure du corps de canule.

3. Procédé selon la revendication 1, dans lequel l'étape d'application d'un revêtement de nitrure de titane sur le corps de canule implique l'application d'un revêtement de nitrure de titane sur une surface extérieure du corps de canule par chauffage indirect par micro-ondes.

4. Procédé selon la revendication 1, dans lequel l'étape d'application d'un revêtement de nitrure de titane sur le corps de canule implique l'application d'un revêtement de nitrure de titane sur une surface extérieure du corps de canule par dépôt en phase vapeur.

5. Procédé selon la revendication 1, dans lequel l'étape de formation d'un biseau sur une partie d'extrémité distale du corps de canule inclut la formation d'un premier bord de coupe le long d'un plan de biseau primaire qui coupe une surface extérieure du corps de canule.

6. Procédé selon la revendication 4, dans lequel l'étape de formation d'un biseau sur une partie d'extrémité distale du corps de canule inclut la formation d'une lancette à une extrémité distale du biseau.

7. Procédé selon la revendication 6, dans lequel l'étape de formation d'une lancette à une extrémité distale du biseau inclut la formation de bords de coupe secondaires le long de plans de biseau secondaires qui coupent une surface extérieure du corps de canule.
